(19) Europäisches Patentamt
European Patent Office
Office européen des brevets

(11) EP 3 761 434 A1

(12) # EUROPEAN PATENT APPLICATION
published in accordance with Art. 153(4) EPC

(43) Date of publication:
**06.01.2021 Bulletin 2021/01**

(21) Application number: **19757215.9**

(22) Date of filing: **22.01.2019**

(51) Int Cl.:
***H01M 10/0567*** *(2010.01)* ***H01M 4/505*** *(2010.01)*
***H01M 4/525*** *(2010.01)* ***H01M 10/052*** *(2010.01)*
***H01M 10/0569*** *(2010.01)*

(86) International application number:
**PCT/JP2019/001805**

(87) International publication number:
**WO 2019/163367 (29.08.2019 Gazette 2019/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.02.2018 JP 2018032472**

(71) Applicant: **Panasonic Intellectual Property Management Co., Ltd.**
**Osaka-shi, Osaka 540-6207 (JP)**

(72) Inventors:
- **ZHONG, Yuanlong**
  **Osaka-shi, Osaka 540-6207 (JP)**
- **HASEGAWA, Masaki**
  **Osaka-shi, Osaka 540-6207 (JP)**

(74) Representative: **Vigand, Philippe et al**
**Novagraaf International SA**
**Chemin de l'Echo 3**
**1213 Onex - Genève (CH)**

(54) **NON-AQUEOUS ELECTROLYTE SECONDARY BATTERY**

(57) Provided is a non-aqueous electrolyte secondary battery comprising: a positive electrode including a positive electrode active material; a negative electrode; and a non-aqueous electrolyte, wherein the positive electrode active material has a composite oxide containing Ni and Li, the proportion of Ni being at least 33 mol% with respect to the total number of moles of metal elements other than Li, and the non-aqueous electrolyte contains: a non-aqueous solvent containing a fluorine-containing cyclic carbonate; an isocyanurate derivative such as triallyl isocyanurate; and a cyclic carboxylic anhydride such as a succinic anhydride.

Figure 1

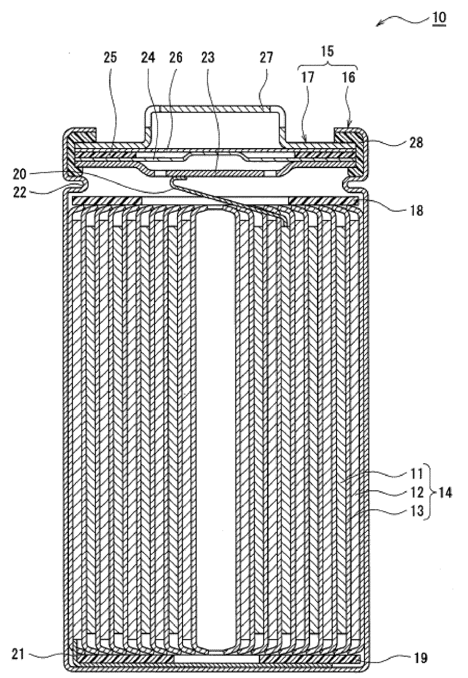


EP 3 761 434 A1

## Description

TECHNICAL FIELD

The present invention relates to a technology for a non-aqueous electrolyte secondary battery.

BACKGROUND ART

Recently, as high output and high energy density secondary batteries, non-aqueous electrolyte secondary batteries comprising a positive electrode, a negative electrode, and a non-aqueous electrolyte have been widely utilized. In the batteries, charging and discharging is conducted by migration of lithium ions or the like between the positive electrode and the negative electrode.

For example, Patent Literature 1 discloses a non-aqueous electrolyte secondary battery comprising a positive electrode, a negative electrode, and an electrolyte solution including a fluorine-containing cyclic carbonate. Patent Literature 1 mentions that use of an electrolyte solution including a fluorine-containing cyclic carbonate improves cycle characteristics at room temperature.

Additionally, Patent Literature 2 mentions that addition of an isocyanate compound such as triallyl isocyanurate to an electrolyte solution including a fluorine-containing cyclic carbonate has an effect of improving the battery performance.

CITATION LIST

PATENT LITERATURE

PATENT LITERATURE 1: Japanese Unexamined Patent Application Publication No. 2013-182807
PATENT LITERATURE 2: Japanese Unexamined Patent Application Publication No. 2014-194930

SUMMARY

However, non-aqueous electrolyte secondary batteries in which an electrolyte solution including a fluorine-containing cyclic carbonate and an isocyanate compound is used have the problem of the deterioration in the battery capacity (initial capacity) and in the charge and the discharge cycle characteristics under a high-temperature environment (e.g., 45°C or more). Particularly, the problem described above become significant in non-aqueous electrolyte secondary batteries in which a positive electrode active material is used, the positive electrode active material including a lithium composite oxide that includes Ni and Li and has a proportion of Ni of 33 mol% or more based on the total number of moles of the metal elements except for Li.

Thus, it is an advantage of the present disclosure to provide a non-aqueous electrolyte secondary battery that can inhibit the deterioration in the battery capacity and in the charge and discharge cycle characteristics under a high-temperature environment even with the use of a positive electrode active material including a composite oxide that includes Ni and Li and has a proportion of Ni of 33 mol% or more based on the total number of moles of the metal elements except for Li.

The non-aqueous electrolyte secondary battery according to one aspect of the present disclosure comprises a positive electrode including a positive electrode active material, a negative electrode, and a non-aqueous electrolyte, the positive electrode active material having a composite oxide that includes Ni and Li and has a proportion of Ni of 33 mol% or more based on the total number of moles of the metal elements except for Li, the non-aqueous electrolyte including:

a non-aqueous solvent including a fluorine-containing cyclic carbonate,
an isocyanuric acid derivative represented by the following formula (1),

[Formula 1]

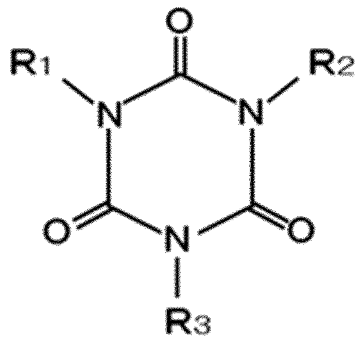

(1)

where $R_1$ is $-C_nH_{2n}-CH=CH_2$, $R_2$ and $R_3$ are each independently H or $-C_nH_{2n}-CH=CH_2$, and n is an integer, and a cyclic carboxylic anhydride represented by the following formula (2),

[Formula 2]

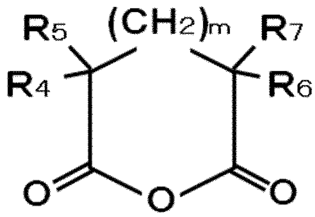

(2)

where $R_4$ to $R_7$ are each independently H, an alkyl group, an alkene group, or an aryl group, and m is 0, 1, or 2.

According to the non-aqueous electrolyte secondary battery according to one aspect of the present disclosure, the deterioration in the battery capacity and in the charge and discharge cycle characteristics under a high-temperature environment can be inhibited even with the use of a positive electrode active material including a composite oxide that includes Ni and Li and has a proportion of Ni of 33 mol% or more based on the total number of moles of the metal elements except for Li.

BRIEF DESCRIPTION OF DRAWING

FIG. 1 is a sectional view of a non-aqueous electrolyte secondary battery, which is one example of the embodiment.

DESCRIPTION OF EMBODIMENTS

(Action of Non-aqueous Electrolyte in Present Disclosure)

In the non-aqueous electrolyte including a fluorine-containing cyclic carbonate, a portion of the fluorine-containing cyclic carbonate is decomposed on the surface of the negative electrode in the initial charging to form a film (SEI film) on the surface of the negative electrode. Usually, formation of an SEI film derived from a fluorine-containing cyclic carbonate inhibits decomposition of the non-aqueous electrolyte that occurs in the subsequent charge and discharge process. However, the SEI film derived from a fluorine-containing cyclic carbonate lacks thermal stability, and thus the SEI film will be broken under a high-temperature environment. As a result, decomposition of the non-aqueous electrolyte component that occurs in the charge and discharge process proceeds, and a side reaction product that prevents the electrode reaction accumulates on the electrode, thereby causing the deterioration in the battery capacity and in the charge and discharge cycle characteristics under a high-temperature environment.

Here, it is considered that allowing an isocyanuric acid derivative, which has been conventionally known to improve the battery performance, to coexist with a fluorine-containing cyclic carbonate can inhibit the deterioration in

the battery capacity and in the charge and discharge cycle characteristics under a high-temperature environment. However, when a positive electrode active material including a composite oxide that includes Ni and Li and has a proportion of Ni of 33 mol% or more based on the total number of moles of the metal elements except for Li is used, the inhibition effect described above still cannot be achieved. It is assumed that this is because a side reaction between Ni and the non-aqueous electrolyte including an isocyanuric acid derivative occurs on the surface of the positive electrode active material and a soluble reaction product produced from the reaction electrophoreses to the negative electrode to thereby denature the film on the surface of the negative electrode.

However, as a result of earnest studies of the present inventors, it has been found that allowing a cyclic carboxylic anhydride and an isocyanuric acid derivative to coexist as described in the following embodiment inhibits the deterioration in the battery capacity and in the charge and discharge cycle characteristics under a high-temperature environment. It is assumed that this is because coexistence of a cyclic carboxylic anhydride and an isocyanuric acid derivative described below has formed a film on the surface of the negative electrode, the film being resistant to a soluble reaction product produced by the above side reaction ascribed to Ni. Specifically, the cyclic carboxylic anhydride absorbs the moisture included in the electrolyte solution. Then, decomposition of the electrolyte salt due to the reaction between the electrolyte salt and the moisture in the electrolyte solution is inhibited, and production of a decomposition product of the electrolyte salt is reduced. As a result, the proportion of the decomposition product of the electrolyte salt included in the film on the surface of this negative electrode is reduced. It is thus assumed that the strength of the film on the surface of the negative electrode is enhanced and the resistance thereof is improved.

Hereinafter, one example of the embodiment will be described in detail. The drawing referred to in the description of the embodiment is drawn schematically, and the dimensional ratio of the constituents drawn in the drawing may be different from that of the actual constituents.

FIG. 1 is a sectional view of a non-aqueous electrolyte secondary battery, which is one example of the embodiment. A non-aqueous electrolyte secondary battery 10 shown in FIG. 1 comprises a wound-type electrode assembly 14 in which a positive electrode 11 and a negative electrode 12 are wound via a separator 13, a non-aqueous electrolyte, insulating plates 18 and 19 each disposed above and under the electrode assembly 14, and a battery case 15 accommodating the members described above. The battery case 15 is composed of a bottomed cylindrical case body 16 and a sealing assembly 17 blocking the opening of the case body 16. Instead of the wound-type electrode assembly 14, an electrode assembly in a different form may be used, such as a layered electrode assembly in which a positive electrode and a negative electrode are alternately layered via a separator. Examples of the battery case 15 can include metal cases such as cylindrical, rectangular, coin-shaped, and button-shaped cases, and resin cases formed by laminating a resin sheet (laminate-type battery).

The case body 16 is, for example, a bottomed cylindrical metal container. A gasket 28 is provided between the case body 16 and the sealing assembly 17 to achieve hermeticity inside the battery. The case body 16 has a projecting portion 22 for supporting the sealing assembly 17, at which a portion of the side wall, for example, projects inside. The projecting portion 22 is preferably formed along the peripheral direction of the case body 16 in a circular shape to support the sealing assembly 17 by the upper surface thereof.

The sealing assembly 17 has a structure in which a filter 23, a lower vent member 24, an insulating member 25, an upper vent member 26, and a cap 27 are layered from the side of the electrode assembly 14. The members constituting the sealing assembly 17 each have, for example, a disk shape or a ring shape, and the members except for the insulating member 25 are each electrically connected to one another. The lower vent member 24 and the upper vent member 26 are connected at each center part to each other, and the insulating member 25 is interposed between the peripheral edges of the vent members. When the internal pressure rises by heat generation due to an internal short circuit or the like, for example, the lower vent member 24 deforms and breaks so as to push up the upper vent member 26 toward the side of the cap 27, and the current path between the lower vent member 24 and the upper vent member 26 is disconnected. When the internal pressure further increases, the upper vent member 26 breaks, and gas is emitted from the opening of the cap 27.

In the non-aqueous electrolyte secondary battery 10 as shown in FIG. 1, a positive electrode lead 20 attached to the positive electrode 11 extends through a through-hole in the insulating plate 18 to the side of the sealing assembly 17, and a negative electrode lead 21 attached to the negative electrode 12 extends through outside the insulating plate 19 to the side of the bottom of the case body 16. The positive electrode lead 20 is connected to the lower surface of the filter 23, which is the bottom plate of the sealing assembly 17, by means of welding or the like. The cap 27, which is the top plate of the sealing assembly 17 electrically connected to the filter 23, serves as the positive electrode terminal. The negative electrode lead 21 is connected to the inner surface of the bottom of the case body 16 by means of welding or the like, and the case body 16 serves as the negative electrode terminal.

The positive electrode, the negative electrode, the separator, and the non-aqueous electrolyte will be described in detail.

[Non-aqueous Electrolyte]

The non-aqueous electrolyte includes a non-aqueous solvent including a fluorine-containing cyclic carbonate, an isocyanuric acid derivative, a cyclic carboxylic anhydride, and an electrolyte salt. The non-aqueous electrolyte is not limited to a liquid electrolyte (non-aqueous electrolyte solution) and may be a solid electrolyte for which a gel-like polymer or the like is used.

The fluorine-containing cyclic carbonate included in the non-aqueous solvent is not particularly limited as long as being a cyclic carbonate containing at least one fluorine. Examples thereof include monofluoroethylene carbonate (FEC), 1,2-difluoroethylene carbonate, 1,2,3-trifluoropropylene carbonate, 2,3-difluoro-2,3-butylene carbonate, and 1,1,1,4,4,4-hexafluoro-2,3-butylene carbonate. Of these, from the viewpoint of suppression of the amount of hydrofluoric acid to be generated under a high-temperature environment and the like, FEC is preferable.

The content of the fluorine-containing cyclic carbonate is, for example, preferably 0.1% by volume or more and 30% by volume or less and more preferably 10% by volume or more and 20% by volume or less based on the total volume of the non-aqueous solvent. When the content of the fluorine-containing cyclic carbonate is less than 0.1% by volume, the amount of an SEI film produced, which is derived from the fluorine-containing cyclic carbonate, is small, and thus, the cycle characteristics at room temperature may deteriorate. When the content of the fluorine-containing cyclic carbonate is more than 30% by volume, the amount of an SEI film produced, which is derived from the fluorine-containing cyclic carbonate, becomes large, and thus, the effect of addition of the isocyanuric acid derivative and the cyclic carboxylic anhydride may not be fully exerted.

The non-aqueous solvent may include, in addition to the fluorine-containing cyclic carbonate, for example, a non-fluorine-based solvent. Examples of the non-fluorine-based solvent can include cyclic carbonates, chain carbonates, carboxylates, cyclic ethers, chain ethers, nitriles such as acetonitrile, amides such as dimethylformamide, and mixed solvents thereof.

Examples of the above cyclic carbonates include ethylene carbonate (EC), propylene carbonate (PC), and butylene carbonate. Examples of the above chain carbonates include dimethyl carbonate, ethylmethyl carbonate (EMC), diethyl carbonate, methylpropyl carbonate, ethylpropyl carbonate, and methylisopropyl carbonate.

Examples of the above carboxylates include methyl acetate, ethyl acetate, propyl acetate, methyl propionate (MP), ethyl propionate, and $\gamma$-butyrolactone.

Examples of the above cyclic ethers include 1,3-dioxolane, 4-methyl-1,3-dioxolane, tetrahydrofuran, 2-methyltetrahydrofuran, propylene oxide, 1,2-butylene oxide, 1,3-dioxane, 1,4-dioxane, 1,3,5-trioxane, furan, 2-methylfuran, 1,8-cineol, and crown ethers.

Examples of the above chain ethers include 1,2-dimethoxyethane, diethyl ether, dipropyl ether, diisopropyl ether, dibutyl ether, dihexyl ether, ethylvinyl ether, butylvinyl ether, methylphenyl ether, ethylphenyl ether, butylphenyl ether, pentylphenyl ether, methoxytoluene, benzylethyl ether, diphenyl ether, dibenzyl ether, o-dimethoxybenzene, 1,2-diethoxyethane, 1,2-dibutoxyethane, diethylene glycol dimethyl ether, diethylene glycol diethyl ether, diethylene glycol dibutyl ether, 1,1-dimethoxymethane, 1,1-diethoxyethane, triethylene glycol dimethyl ether, and tetraethylene glycol dimethyl ether.

The isocyanuric acid derivative included in the non-aqueous electrolyte is represented by the following formula:

[Formula 3]

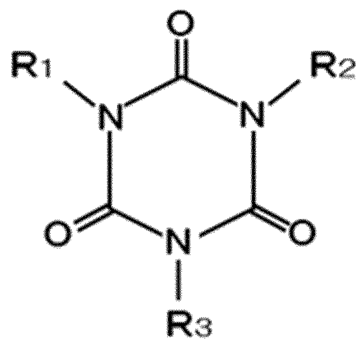

where $R_1$ is $-C_nH_{2n}-CH=CH_2$, $R_2$ and $R_3$ are each independently H or $-C_nH_{2n}-CH=CH_2$, and n is an integer, preferably 1 to 20, for example. It is considered that the isocyanuric acid and cyclic carboxylic anhydride form a robust film on the negative electrode, and further, it is assumed that the presence of a double bond on at least one of $R_1$ to $R_3$ enhances the molecular weight of the film to thereby form a more robust film.

The isocyanuric acid derivative included in the non-aqueous electrolyte is not particularly limited as long as the derivative is a substance represented by the above formula. Examples thereof include triallyl isocyanurate, tributenyl isocyanurate, tripentenyl isocyanurate, and trihexenyl isocyanurate. Among these, triallyl isocyanurate is preferable in view of, for example, inhibiting the deterioration in the battery capacity and in the charge and discharge cycle characteristics under a high-temperature environment. Triallyl isocyanurate is represented by the following structural formula.

[Formula 4]

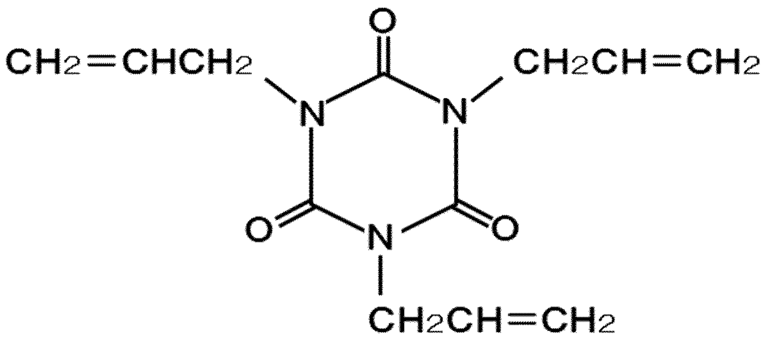

The content of the isocyanuric acid derivative included in the non-aqueous electrolyte is, for example, preferably in the range of 0.1% by mass or more and 1.5% by mass or less, more preferably in the range of 0.25% by mass or more and 1% by mass or less, and even more preferably in the range of 0.25% by mass or more and 0.5% by mass or less based on the total mass of the non-aqueous electrolyte in view of, for example, inhibiting the deterioration in the battery capacity and in the charge and discharge cycle characteristics under a high-temperature environment. When the content is less than 0.1% by mass, the effect of inhibiting the deterioration in the charge and discharge cycle characteristics under a high-temperature environment may be lessened. When the content exceeds 1.5% by weight, the effect of inhibiting the deterioration in the battery capacity under a high-temperature environment may be lessened. It is considered that this is because film formation on the active material surface becomes excessive to prevent occlusion and releasing of lithium ions in the active material and thus the battery capacity deteriorates.

The cyclic carboxylic anhydride included in the non-aqueous electrolyte is represented by the following formula:

[Formula 5]

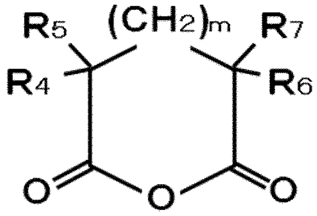

where $R_4$ to $R_7$ are each independently hydrogen, an alkyl group, an alkene group, or an aryl group, and m is 0, 1, or 2.

The cyclic carboxylic anhydride included in the non-aqueous electrolyte is not particularly limited as long as the being a substance represented by the above formula. In view of, for example, inhibiting the deterioration in the battery capacity and in the charge and discharge cycle characteristics under a high-temperature environment, at least one of the group consisting of succinic anhydride and glutaric anhydride is preferably included. Among these, succinic anhydride is preferable in view of, for example, inhibiting gas generation under a high-temperature environment.

Succinic anhydride is represented by the following structural formula.

[Formula 6]

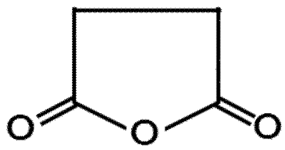

Glutaric anhydride is represented by the following structural formula.

[Formula 7]

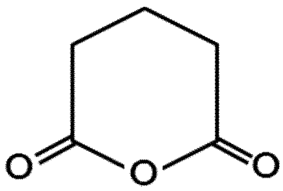

The content of the cyclic carboxylic anhydride included in the non-aqueous electrolyte is, for example, preferably in the range of 0.1% by mass or more and 1.5% by mass or less, more preferably in the range of 0.25% by mass or more and 1% by mass or less, and more preferably in the range of 0.25% by mass or more and 0.75% by mass or less based on the total mass of the non-aqueous electrolyte in view of, for example, inhibiting the deterioration in the battery capacity and in the charge and discharge cycle characteristics under a high-temperature environment. When the content is less than 0.1% by mass, the effect of inhibiting the deterioration in the charge and discharge cycle characteristics under a high-temperature environment may be lessened. When the content exceeds 1.5% by weight, the effect of inhibiting the deterioration in the battery capacity under a high-temperature environment may be lessened.

The electrolyte salt included in the non-aqueous electrolyte is preferably a lithium salt. As the lithium salt, ones commonly used as a supporting salt in conventional non-aqueous electrolyte secondary batteries can be used. Specific examples thereof include $LiBF_4$, $LiClO_4$, $LiPF_6$, $LiAsF_6$, $LiSbF_6$, $LiAlCl_4$, LiSCN, $LiCF_3SO_3$, $LiCF_3CO_2$, $Li(P(C_2O_4)F_4)$, $LiPF_{6-x}(C_nF_{2n+1})_x$ (where 1 < x < 6, n is 1 or 2), $LiB_{10}Cl10$, LiCl, LiBr, LiI, chloroborane lithium, lithium lower aliphatic carboxylate, borates such as $Li_2B_4O_7$ and $Li(B(C_2O_4)F_2)$, and imide salts such as $LiN(SO_2CF_3)_2$ and $LiN(C_lF_{2l+1}SO_2)(C_mF_{2m+1}SO_2)$ (where 1 and m are each an integer of 0 or more). One of these lithium salts may be used alone, or a plurality thereof may be used in admixture. Of these, $LiPF_6$ is preferably used from the viewpoint of ionic conductive properties, electrochemical stability, and the like. The concentration of the lithium salt is preferably 0.8 to 1.8 mol per L of the non-aqueous solvent.

[Positive Electrode]

The positive electrode 11 is composed of, for example, a positive electrode current collector and a positive electrode active material layer formed on the positive electrode current collector. As the positive electrode current collector, it is possible to use foil of a metal stable in the potential range of the positive electrode, such as aluminum, a film including such a metal disposed on the surface layer thereof, or the like. The positive electrode 11 can be produced by, for example, coating a positive electrode mixture slurry including a positive electrode active material, a binder, and the like onto a positive electrode current collector, drying the coated film, and then rolling the coated film to form a positive electrode active material layer on the positive electrode current collector.

The positive electrode active material layer includes a positive electrode active material. The positive electrode active material layer preferably includes a positive electrode active material as well as a binder and a conductive agent.

The positive electrode active material includes a composite oxide that includes Ni and Li and has a proportion of Ni of 33 mol% or more based on the total number of moles of the metal elements except for Li (hereinafter, simply referred to as the composite oxide). In the positive electrode active material including a composite oxide having a proportion of Ni of 33 mol% or more, as previously mentioned, a film of a side reaction product ascribed to an oxide of nickel is produced and grown, and thus, the battery capacity and charge and discharge cycle characteristics tend to deteriorate under a high-temperature environment. However, as in the present embodiment, applying an electrolyte solution in which the isocyanuric acid derivative and cyclic carboxylic anhydride coexist can inhibit the deterioration in

the battery capacity and in the charge and discharge cycle characteristics under a high-temperature environment. The content of the composite oxide particles is, for example, preferably 50% by mass or more and more preferably 80% by mass or more based on the total mass of the positive electrode active material.

The proportion of Ni in the composite oxide is only required to be 33 mol% or more, and, in view of, for example, obtaining a larger capacity of the battery, preferably 75 mol% or more and more preferably 80 mol% or more, based on the total number of moles of the metal elements except for lithium.

The composite oxide is preferably, for example, a composite oxide represented by the general formula: $Li_xNi_yM_{(1-y)}O_2$, where $0.1 \leq x \leq 1.2$, $0.33 \leq y \leq 1$, and M is at least one metal element. Examples of the metal element M include Co, Mn, Mg, Zr, Al, Cr, V, Ce, Ti, Fe, K, Ga, and In. The metal element M preferably includes, among these, at least one of the group consisting of cobalt (Co), manganese (Mn), and aluminum (Al). The metal element M particularly preferably includes Co and Al from the viewpoint of the battery capacity and the like.

The positive electrode active material may include a lithium composite oxide other than the above composite oxides, and examples thereof include non Ni-containing composite oxide such as $LiCoO_2$ and $LiMn_2O_4$ and composite oxides in which the proportion of Ni is less than 33 mol% based on the total number of moles of the metal elements except for Li.

As the conductive agent, carbon powders such as carbon black, acetylene black, Ketjen black, and graphite may be used singly or in combinations of two or more thereof.

Examples of the binder include fluorine-based polymers and rubber-based polymers. Examples of the fluorine-based polymer include polytetrafluoroethylene (PTFE), polyvinylidene fluoride (PVdF), and modifications thereof, and examples of the rubber-based polymer include ethylene-propylene-isoprene copolymers and ethylene-propylene-butadiene copolymers. These may be used singly or in combinations of two or more thereof.

[Negative Electrode]

The negative electrode 12 comprises, for example, a negative electrode current collector and a negative electrode active material layer formed on the negative electrode current collector. As the negative electrode current collector, it is possible to use foil of a metal stable in the potential range of the negative electrode, such as copper, a film including such a metal disposed on the surface layer thereof, or the like. The negative electrode active material layer preferably includes a negative electrode active material and includes a thickener and a binder additionally. The negative electrode 12 can be produced by, for example, coating a negative electrode mixture slurry in which a negative electrode active material, a thickener, and a binder are dispersed at a predetermined weight ratio in water onto a negative electrode current collector, drying the coated film, and then rolling the coated film to form a negative electrode active material layer on the negative electrode current collector.

Examples of the negative electrode active material include carbon materials and non-carbon materials capable of occluding and releasing lithium ions. Examples of the carbon material include graphite, non-graphitizable carbon, graphitizable carbon, fibrous carbon, cokes, and carbon black. Examples of the non-carbon material include silicon, tin, and alloys and oxides mainly based thereon.

As the binder, a fluorine-based polymer, a rubber-based polymer, or the like can be used as in the case of the positive electrode, and a styrene-butadiene copolymer (SBR) or a modification thereof may be used. Examples of the thickener include carboxymethyl cellulose (CMC) and polyethylene oxide (PEO). These may be used singly or in combinations of two or more thereof.

[Separator]

As the separator 13, for example, a porous sheet having ion permeability and insulation is preferably used. Specific examples of the porous sheet include microporous thin films, woven fabric, and non-woven fabric. As materials for the separator, olefin resins such as polyethylene and polypropylene, cellulose, and the like are suitable. The separator may be a laminate having a cellulosic fiber layer and a layer of a thermoplastic resin fiber such as an olefinic resin. The separator may be a multi-layer separator including a polyethylene layer and a polypropylene layer, or a separator including a material such as an aramid-based resin, ceramic, or the like applied on the surface thereof may be used.

EXAMPLES

Hereinbelow, the present disclosure will be further described with reference to examples, but the present disclosure is not intended to be limited to the following examples.

<Example 1>

[Production of Positive Electrode]

As a positive electrode active material, a lithium composite oxide represented by the general formula: $LiNi_{0.8}Co_{0.15}Al_{0.05}O_2$ was used. The positive electrode active material at 95% by mass, acetylene black as a conductive agent at 3% by mass, and polyvinylidene fluoride as a binder at 2% by mass were mixed, and N-methyl-2-pyrrolidone (NMP) was added thereto to prepare a positive electrode mixture slurry. Subsequently, the positive electrode mixture slurry was coated onto both the surfaces of a positive electrode current collector made of aluminum of 15 μm in thickness by a doctor blade method, and the coated films were rolled to form a positive electrode active material layer of 70 μm in thickness on both the surfaces of the positive electrode current collector. This was used as a positive electrode.

[Preparation of Negative Electrode]

Graphite as a negative electrode active material at 97% by mass, carboxymethyl cellulose (CMC) as a thickener at 2% by mass, a styrene-butadiene copolymer (SBR) as a binder at 1% by mass were mixed, and water was added thereto to prepare a negative electrode mixture slurry. Subsequently, the negative electrode mixture slurry was coated on both the surfaces of a negative electrode current collector made of copper of 10 μm in thickness by a doctor blade method, and the coated films were rolled to form a negative electrode active material layer of 100 μm in thickness on both the surfaces of the negative electrode current collector. This was used as a negative electrode.

[Preparation of Electrolyte Solution]

To a mixed solvent prepared by mixing fluorinated ethylene carbonate (FEC) and dimethyl carbonate (DMC) at a volume ratio of 20:80, $LiPF_6$ was dissolved at a concentration of 1 mol/L, and additionally, triallyl isocyanurate at 0.5% by mass and succinic anhydride at 0.5% by mass were dissolved thereto to prepare an electrolyte solution.

[Production of Non-aqueous Electrolyte Secondary Battery]

The positive electrode and the negative electrode described above were each cut into a predetermined size. The cut electrodes were each fitted with an electrode tab and wound via a separator to produce a wound-type electrode assembly. Next, while insulating plates were each disposed above and under the electrode assembly, the electrode assembly was housed in an exterior can made of Ni-plated steel and having a diameter of 18 mm and a height of 65 mm. The negative electrode tab was welded on the inner bottom of the battery exterior can as well as the positive electrode tab was welded on the bottom plate portion of the sealing assembly. Then, the above electrolyte solution was injected from the opening of the exterior can, and the exterior can was sealed with the sealing assembly to produce a non-aqueous electrolyte secondary battery.

<Example 2>

A non-aqueous electrolyte secondary battery was produced in the same manner as in Example 1 except that succinic anhydride was replaced by glutaric anhydride in the preparation of an electrolyte solution.

<Comparative Example 1>

A non-aqueous electrolyte secondary battery was produced in the same manner as in Example 1 except that triallyl isocyanurate and succinic anhydride were not added in the preparation of an electrolyte solution.

<Comparative Example 2>

A non-aqueous electrolyte secondary battery was produced in the same manner as in Example 1 except that succinic anhydride was not added in the preparation of an electrolyte solution.

[High Temperature Charge/Discharge Test]

Each of the batteries according to Examples and Comparative Examples was subjected to one cycle of charge and discharge at a charge end voltage of 4.1 V and a discharge end voltage of 3.0 V with a charge/discharge current corresponding to 0.5 It under a temperature condition of 45°C. Note that 1 It is the current value at which the rated

capacity of the battery is discharged in an hour. The difference in the discharge capacity of each of the batteries of Examples 1 and 2 and Comparative Example 2 was calculated on the basis of the discharge capacity of the battery of Comparative Example 1. The results are shown in Table 1.

[High Temperature Cyclic Test]

Each of the batteries according to Examples and Comparative Examples was subjected to 100 cycles of charge and discharge at a charge end voltage of 4.1 V and a discharge end voltage of 3.0 V with a charge/discharge current corresponding to 0.5 It under a high-temperature environment of 45°C. The reduction in the discharge capacity after 100 cycles was calculated (discharge capacity at first cycle - discharge capacity at 100th cycle). The results are shown in Table 1.

[Amount of Change in Average Operational Voltage]

In each battery, the average operational voltage at the first cycle was subtracted from the average operational voltage at the 100th cycle in the above high temperature cyclic test to determine the amount of change in the average operational voltage of each battery. The results are shown in Table 1. The average operational voltage is determined by the following formula.

$$\text{Average operational voltage (mV)} = \text{discharge capacity (Wh)} / \text{discharge capacity (Ah)}$$

[High Temperature Storage Test]

Each of the batteries charged to a charge end voltage of 4.1 V with a charge/discharge current corresponding to 0.5 It was stored in a thermostatic chamber at 80°C for three days. Thereafter, the amount of gas in the battery was measured with gas chromatography, and the amount of gas measured was taken as the amount of gas generated after high temperature storage. In Table 1, the amount of gas generated after high temperature storage of each of the batteries of Examples 1 and 2 and Comparative Example 2 is shown relatively on the basis of the amount of gas generated after high temperature storage of the battery of Comparative Example 1 (100%).

[Table 1]

| | Non-aqueous electrolyte | | High temperature test | | | |
|---|---|---|---|---|---|---|
| | Substance added 1 (% by mass) | Substance added 2 (% by mass) | Difference in discharge capacity | Reduction in capacity after 100 cycles | Amount of change in average operational voltage after 100 cycles | Amount of gas generated |
| Example 1 | Succinic anhydride (0.5) | Triallyl isocyanurate (0.5) | 4mAh | -128mAh | 2.3mV | 48% |
| Example 2 | Glutaric anhydride (0.5) | Triallyl isocyanurate (0.5) | 18mAh | -128mAh | 3.0mV | 65% |
| Comparative Example 1 | - | - | - | -174mAh | -1.3mV | 100% |
| Comparative Example 2 | | Triallyl isocyanurate (0.5) | -13mAh | -161mAh | 1.7mV | 51% |

In the batteries of Examples 1 and 2, the discharge capacity increased under a high-temperature environment and the deterioration in the charge and discharge cycle characteristics was inhibited, in comparison with the batteries of Comparative Examples 1 and 2. It can be said from these results that addition of both the above isocyanuric acid

derivative and cyclic carboxylic anhydride into the electrolyte solution can inhibit the deterioration in the battery capacity and in the charge and discharge cycle characteristics under a high-temperature environment in a non-aqueous electrolyte secondary battery in which a positive electrode active material having a composite oxide that includes Ni and Li and has a proportion of Ni of 33 mol% or more based on the total number of moles of the metal elements except for Li is used.

As can be seen from the results of the amount of change in the average operational voltage in Table 1, in the batteries of Examples 1 and 2, the average operational voltage at the 100th cycle increased more than the average operational voltage at the first cycle. Meanwhile, in the battery of Comparative Example 1, the average operational voltage at the 100th cycle became lower than the average operational voltage at the first cycle. A decrease in the average operational voltage of the battery means an increase in the resistance of the battery. Thus, it can be said that an increase in the battery resistance under a high-temperature environment in the batteries of Examples 1 and 2 was inhibited more than in the battery of Comparative Example 1.

Among the batteries of Examples 1 and 2, the amount of gas generated under a high-temperature environment was inhibited more in Example 1, in which succinic anhydride was used as the cyclic carboxylic anhydride, than in Example 2, in which glutaric anhydride was used.

REFERENCE SIGNS LIST

10 non-aqueous electrolyte secondary battery
11 positive electrode
12 negative electrode
13 separator
14 electrode assembly
15 battery case
16 case body
17 sealing assembly
18, 19 insulating plate
20 positive electrode lead
21 negative electrode lead
22 projecting portion
23 filter
24 lower vent member
25 insulating member
26 upper vent member
27 cap
28 gasket

## Claims

**1.** A non-aqueous electrolyte secondary battery, comprising:

a positive electrode including a positive electrode active material;
a negative electrode; and
a non-aqueous electrolyte, wherein
the positive electrode active material has a composite oxide that includes Ni and Li and has a proportion of Ni of 33 mol% or more based on the total number of moles of the metal elements except for Li,
the non-aqueous electrolyte includes:

a non-aqueous solvent including a fluorine-containing cyclic carbonate,
an isocyanuric acid derivative represented by the following formula (1):

[Formula 1]

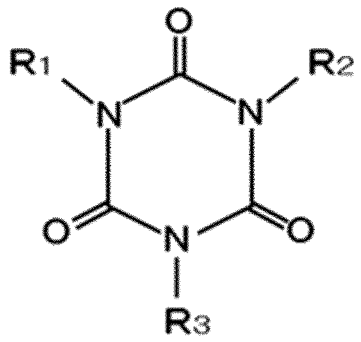

(1)

where $R_1$ is $-C_nH_{2n}-CH=CH_2$, $R_2$ and $R_3$ are each independently H or $-C_nH_{2n}-CH=CH_2$, and n is an integer, and a cyclic carboxylic anhydride represented by the following formula (2):

[Formula 2]

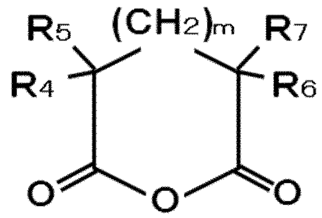

(2)

where $R_4$ to $R_7$ are each independently H, an alkyl group, an alkene group, or an aryl group, and m is 0, 1, or 2.

**2.** The non-aqueous electrolyte secondary battery according to claim 1, wherein the composite oxide has a proportion of Ni of 75 mol% or more based on the total number of moles of the metal elements except for Li.

**3.** The non-aqueous electrolyte secondary battery according to claim 1 or 2, wherein a content of the fluorine-containing cyclic carbonate is 0.1% by volume or more and 30% by volume or less based on the total volume of the non-aqueous solvent.

**4.** The non-aqueous electrolyte secondary battery according to any one of claims 1 to 3, wherein a content of the cyclic carboxylic anhydride is 0.1% by mass or more and 1.5% by mass or less based on the total mass of the non-aqueous electrolyte.

**5.** The non-aqueous electrolyte secondary battery according to any one of claims 1 to 4, wherein a content of the isocyanuric acid derivative is 0.1% by mass or more and 1.5% by mass or less based on the total mass of the non-aqueous electrolyte.

**6.** The non-aqueous electrolyte secondary battery according to any one of claims 1 to 5, wherein the cyclic carboxylic anhydride includes at least one of the group consisting of succinic anhydride and glutaric anhydride.

**7.** The non-aqueous electrolyte secondary battery according to any one of claims 1 to 6, wherein the isocyanuric acid derivative includes triallyl isocyanurate.

**8.** The non-aqueous electrolyte secondary battery according to any one of claims 1 to 7, wherein the composite oxide includes at least one of the group consisting of Co, Al, and Mn.

Figure 1

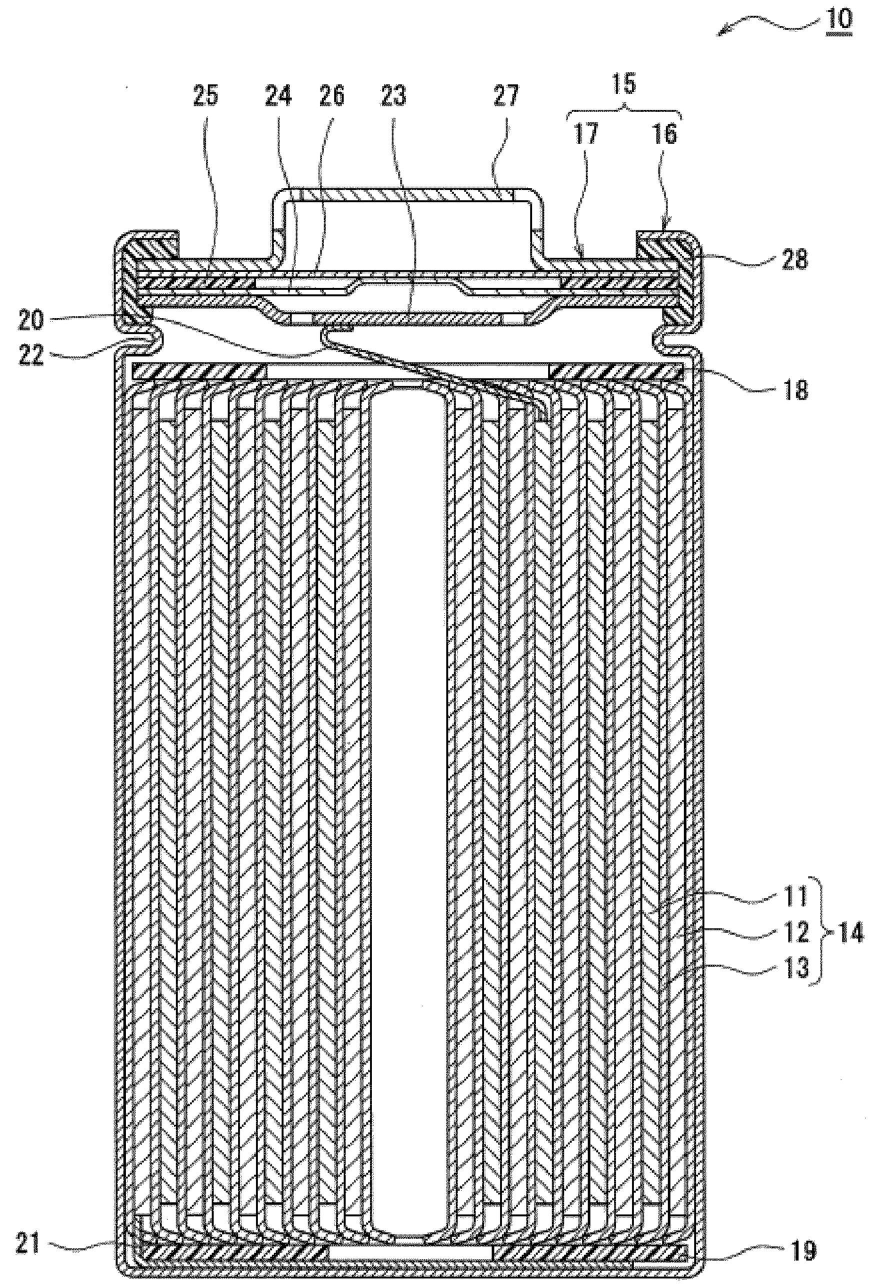

**INTERNATIONAL SEARCH REPORT**

International application No.
PCT/JP2019/001805

A. CLASSIFICATION OF SUBJECT MATTER
Int.Cl. H01M10/0567(2010.01)i, H01M4/505(2010.01)i, H01M4/525(2010.01)i, H01M10/052(2010.01)i, H01M10/0569(2010.01)i

According to International Patent Classification (IPC) or to both national classification and IPC

B. FIELDS SEARCHED

Minimum documentation searched (classification system followed by classification symbols)
Int.Cl. H01M10/0567, H01M4/505, H01M4/525, H01M10/052, H01M10/0569

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | WO 2014/133107 A1 (MITSUBISHI CHEMICAL CORP.) 04 September 2014, claims, paragraphs [0159], [0251], [0396]-[0440], examples 3-A-7, 3-A-8, 4-6, 4-7 & US 2015/0364794 A1 claims, paragraphs [0242], [0435], [0712]-[0779], examples 3-A-7, 3-A-8, 4-6, 4-7 & CN 104995784 A & KR 10-2015-0125928 A | 1-8 |
| A | JP 2014-63733 A (MITSUBISHI CHEMICAL CORP.) 10 April 2014, entire text (Family: none) | 1-8 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 April 2019 (08.04.2019) | 16 April 2019 (16.04.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013182807 A
- JP 2014194930 A